# EUROPEAN PATENT SPECIFICATION

(11) **EP 1 948 217 B1**
(45) Date of publication and mention of the grant of the patent: **04.01.2012**
(21) Application number: 06780613.3
(22) Date of filing: 11.08.2006
(51) Int. Cl.: A61K 38/18, A61P 25/00

(54) **USE OF NERVE GROWTH FACTOR IN EYE-DROPS FOR THERAPY OF PATHOLOGIES OF THE CENTRAL NERVOUS SYSTEM, SUCH AS ALZHEIMER'S AND PARKINSON'S DISEASE**
VERWENDUNG DES NERVENWACHSTUMFAKTORS IN AUGENTROPFEN ZUR BEHANDLUNG VON ERKRANKUNGEN DES ZENTRALEN NERVENSYSTEMS, WIE Z.B. ALZHEIMER-KRANKHEIT UND PARKINSON-KRANKHEIT
UTILISATION DU FACTEUR DE CROISSANCE DU NERF DANS DES GOUTTES OPHTALMIQUES POUR LE TRAITEMENT DE PATHOLOGIES DU SYSTEME NERVEUX CENTRAL TELLES QUE LA MALADIE D'ALZHEIMER ET LA MALADIE DE PARKINSON

(30) Priority: 19.08.2005 IT RM20050447
(43) Date of publication of application: 30.07.2008
(73) Proprietor: Anabasis s.r.l., 00172 Roma (IT)
(72) Inventor: LAMBIASE, Alessandro, I-00172 Roma (IT); BONINI, Stefano, I-00172 Roma (IT)
(74) Representative: Teuten, Andrew John
(86) International application number: PCT/IT2006/000620
(87) International publication number: WO 2007/020672

(56) References cited:
- WO-A-00/44396
- WO-A-98/48002
- US-A1- 2002 009 498
- US-A1- 2003 181 354
- DATABASE WPI Week 200017 Derwent Publications Ltd., London, GB; AN 2000-195449 XP002417034 & WO 00/07613 A1 (ADVANCED MEDICINE RES INST) 17 February 2000 (2000-02-17)

## Description

The present invention concerns the use of nerve growth factor in eye-drops for the therapy of pathologies of the central nervous system, such as Alzheimer's Disease and Parkinson's Disease. More specifically, the invention concerns the use of the neurotrophin called nerve growth factor (NGF) for the treatment of pathologies affecting encephalic structures, such as the hippocampus, cerebral cortex, basal forebrain, medial septum, Broca's diagonal band, Meynert's basal nucleus, substantia nigra pars compacta, striatum and cerebellum, by a simple topical administration over the ocular surface, e.g. in the form of eye-drops or ophthalmic ointment.

Nerve growth factor (NGF) is a chief molecule of a complex neurotrophin family, and is well known for its trophic, tropic and differentiating activity on cholinergic neurons of the central nervous system and on the peripheral nervous system. NGF is produced by many mammalian tissues, including man, and is released in the bloodstream in greater quantities during the growth and differentiation of the nervous system. Biological, biochemical and molecular studies carried out on *in vitro* cell systems have highlighted a very high homology between murine and human NGF. Moreover, in man as in many other animal species, NGF is normally present both in the cerebrospinal fluid and in the bloodstream at concentrations of 10-50 pg/ml, which increase in some inflammatory pathologies (autoimmune and allergic diseases, etc.) and decrease in others (diabetes).

NGF was discovered by Prof Rita Levi-Montalcini, in the Zoology Institute of Washington University of St. Louis (Levi-Montalcini R., Harvey Lect., 60:217, 1966), and represents an important step in the study of nerve cell growth and differentiation mechanisms, as it is is able to influence the development and preservation of the biological functions and regeneration of neurons. Prof R. Levi-Montalcini was awarded the Nobel Prize for Medicine and Physiology in 1986 for discovering this molecule and for characterising its biological role both in the peripheral and central nervous system.

Many *in vitro* and *in vivo* experimental studies have demonstrated the physiopathological importance of NGF in preventing neuronal damage of a surgical, chemical, mechanical and ischemic origin, thus making it the ideal candidate for use in the therapy of many pathologies of the central and peripheral nervous system (Hefti F., J. Neurobiol., 25:1418, 1994; Fricker J. Lancet, 349:480, 1997). In fact, for some years now, clinical trials have been carried out on patients suffering from Parkinson's Disease and Alzheimer's Disease by intracerebral administration of murine NGF (see, for example, Olson L. et al., J. Neural Trans.: Parkinson's Disease and Dementia Section, 4: 79, 1992). The results of these studies have confirmed the observations made on animal models and have highlighted the absence of possible side effects following the administration of murine NGF. This characteristic was later confirmed for recombinant human NGF (Petty B.G. et al., Annals of Neurology, 36:244-246, 1994).

Because, since its discovery, studies on the characterisation of the biological, biochemical, molecular, preclinical and clinical effects of NGF have been carried out almost exclusively with NGF isolated from submandibular glands of adult rodents, the greatest amount of acquired data currently concerns murine NGF. The biochemical properties of murine NGF have been described, in particular, in a work dating back to 1968 (Levi-Montalcini R. & Angeletti P.U., Physiological Reviews, 48:534, 1968).

The NGF contained in murine salivary glands is a 140 kdalton molecular complex with a sedimentation coefficient of 7S, and consists of three subunits, α, β and γ - the second of which represents the actual active form. The latter, known as βNGF, has a sedimentation coefficient of 2.5S and is normally extracted and purified according to three not very different methodologies (Bocchini V., Angeletti P.U., Biochemistry, 64:787-793, 1969; Varon S. et al., Methods in Neurochemistry, 203-229, 1972; Mobley W.C. et al., Molecular Brain Research, 387: 53-62, 1986).

The βNGF obtained in this way is a dimer of about 13,000 dalton composed of two identical chains of 118 amino acids. Each chain is stabilised by three disulphide bridges, while non-covalent bonds assure the stabilisation of the dimeric structure. The molecule is very stable and is soluble in nearly all solvents, both aqueous and oily, and keeps its biochemical characteristics and biological activity unchanged. Further details on the structure and on the physical and biochemical properties of the molecule are reported in Greene, L.A. & Shooter, E.M., Ann. Rev. Neurosci. 3:353, 1980.

The structure of βNGF was recently further clarified by means of crystallographic analysis. The analysis revealed the presence of three antiparallel pairs of filaments with a secondary structure of the α2 type, enabling the formation of a flat surface along which the two chains join together to yield the active dimer. On these βNGF chains the presence of four "loop" regions has been found, wherein many variable amino acids are included. These variable amino acids are probably responsible for receptor recognition specificity.

The biological effect of NGF is mediated by two receptors present on the surface of the corresponding target cells. The existence of many antibodies that selectively inhibit the biological effect of NGF has enabled an accurate characterisation and modulation of its activity, both in cellular systems and *in vivo.*

More recently, human NGF has been synthesised by means of genetic engineering techniques (Iwane, M. et al., Biochem. Biophys. Res. Commun., 171:116, 1990), and small amounts of human NGF are now commercially available, too. However, direct experimentation has shown that the biological activity of human NGF is very low compared to murine NGF activity. Moreover, it must be borne in mind that almost all the currently available data on man - both *in vitro* and *in vivo -* have been obtained by using murine NGF, and no undesirable side effects resulting from murine origin of the molecule have ever been found.

Studies carried out on animal models since the 1970s have suggested a possible involvement of NGF in pathologies concerning the central nervous system. NGF involvement in pathological mechanisms of degenerative pathologies of the central nervous system was suspected about 30 years ago by observing that NGF combats the atrophy of cholinergic neurons in the basal forebrain and the resulting cognitive deficiencies in old and injured rats (Hefti F., J. Neurosci. 6(8):2155-62, 1986). In particular, in recent years many experimental confirmations have pointed to a consistent correlation between a lack of endogenous synthesis of the NGF molecule and the development of some specific pathologies of the central nervous system (CNS), peripheral nervous system (PNS) and skin diseases (Tuszynski M.H. et al., J. Mol. Neurosci., 19:207, 2002; Nakagawara A. et al., N. Engl. J. Med., 328:847-854, 1993; De Santis S. et al., Clin. Cancer Res. 6: 90-95, 2000; Kaye D.M. et al., Circ. Res., 86:e80-e84, 2000; Villoslada P. et al., J. Exp. Med., 191:1799-1806, 2000; Salvinelli F. et al., J. Biol. Regul. Homeost. Agents, 16:176-80, 2002).

Starting from these observations, some researchers started up studies focusing on the therapeutic use of NGF in human lining tissue pathologies, such as corneal ulcers, ulcers from diabetes or decubitus, and the vasculitis associated with rheumatoid arthritis, by means of the topical administration of NGF (Costa N. et al., Ann. Ist. Super. Sanità, 38:187-194, 2002; Aloe, L. e Calzà, L. eds., Prog. Brain Res., 146:1-544, 2004; Lambiase A. et al., New Engl. J. Med., 338: 1174-1180, 1998; Bernabei R. et al., The Lancet, 354:182, 1999; Lambiase A. et al., Arch. Ophthalmol., 118:1446-1449, 2000; Tuveri M. et al., The Lancet, 356:1739-1740, 2000; Chiaretti A. et al., Arch. Dis. Child., 87:446-48, 2002; Landi F. et al., Ann. Int. Medicine, 139:635-641, 2003; Generini S. et al., Exp. Clin. Endocrinol. Diabetes, 112:542-4, 2004; Aloe L., Progr. Brain Res., 146:279-89, 2004). Unfortunately, despite the progress achieved for these surface pathologies, NGF is still unused in the treatment of disorders of the central nervous system notwithstanding the great evidence that exists on the involvement of this neurotrophin in the pathogenesis of neurodegenerative diseases.

As is known, Alzheimer's Disease (AD) is a neurodegenerative disease in the elderly that leads to the gradual loss of memory and of the main cognitive abilities (Selkoe D.J., Physiol. Rev., 81(2):741-66, 2001). There is a sporadic form of this disease (concerning over 95% of the patients affected) and a familiar form (under 5% of the patients affected), linked to gene mutations due to APP (Amyloid Precursor Protein), PS1 (Presenilin1) and PS2 (Presenilin2). In both forms of the disease the cognitive decline is accompanied by neuronal death, the loss of synapses and the forming of intracellular neurofibrillary tangles consisting of the protein cytoskeleton tau present in a hyperphosphorylated form and arranged in molecular aggregations called PHF (Paired Helical Filaments). Another histopathological sign of Alzheimer's Disease (AD) is the extracellular accumulation of amyloid protein (amyloid β-peptide, Aβ), a peptide produced by the pathological proteolysis of APP. This accumulation goes to make up what is currently known as amyloid plaques or senile plaques. These cell disorders are mainly found in the hippocampus and cerebral cortex, while in the basal forebrain and Meynert's basal nucleus, there is a decrease in the number of cholinergic cells (Saper C.B. et al., Neurology, 35(8):1089-95, 1985; Palmer A.M., Neurodegeneration, 5(4):381-91, 1996; Mufson E.J. et al., J. Chem. Neuroanat., 26(4):233-42, 2003).

This particular aspect of AD has led to the formulation of current therapies involving the use of cholinesterase inhibiting agents (and thus promoting the restoring of normal "cholinergic" functioning). It must be stressed that the effects of all the currently available anti-AD drugs are definitely insufficient since their action is limited to a partial slowing down of the disease, and on the condition that the treatment begins at a sufficiently early stage of the disease.

Parkinson's Disease (PD) is the second most frequent neurodegenerative pathology (after AD) in the population and is characterised, from an anatomical-pathological standpoint, by a selective degeneration of the substance known as nigra pars compacta - a nucleus populated by dopaminergic neurons containing melanin, that send their own projections to the striatum. The progressive degeneration of this neuronal population and the resulting dopaminergic denervation of the striatum cause a cascade of alterations disrupting the functional architecture of the base nuclei, leading to the occurrence of the motor symptoms of PD.

Despite the fact that over 40 years have gone by since the nigrostriatal deficit of dopamine was first identified as the neurotransmitter alteration responsible for Parkinson symptomatology, the ethiopathogenesis and physiopathology of PD continue to present several little known areas. This has so far meant a lack of valid therapeutic alternatives to L-DOPA (or levodopa), the precursor of dopamine, still considered to be the "golden standard" in the treatment of PD.

Studies carried out over the last twenty years on in vivo and *in vitro* experimental models have provided precious information, enabling the experimentation of new therapeutic strategies, some of which - such as "deep brain stimulation" (DBS) - have been successfully incorporated in clinical practice. However, even the most recent therapeutic approaches have not been able to supplant levodopa from its position of eminence, despite the undesirable effects (dyskinesia and motor fluctuations, in particular) that inevitably accompany the protracted intake of this drug over a period of years. The most serious deficit at present is the lack of therapeutic tools for dealing with the disease not so much, or not just, from a symptomatic standpoint, as with the case of L-DOPA, but also for enabling a containment of the neurodegenerative process, if not downright for triggering regenerative mechanisms at the nigrostriatal level. In other words, a real leap forward in the treatment of PD can be achieved only when therapeutic strategies actually involving neuroprotective and/or neuroreparatory processes are devised.

Turning back to nerve growth factor, the main obstacle to its use in these therapies lies in the fact that concentrations of such magnitude have to be conveyed to the brain tissues that they cannot be taken via the normal administration methods. In fact, all the currently conducted studies have shown NGF effectiveness only if administered intracerebrally (intracerebroventricularly), since this molecule is unable to pass through the blood-brain barrier in therapeutic concentrations via systemic administration. The most recent therapeutic strategy for NGF use as an anti-AD drug is based on the intracerebral inoculation of genetically modified cells such that they locally release NGF (Tuszynski M.H. et al., J. Mol. Neurosci., 19(1-2):207, 2002; Tuszynski M.H. et al., Nat. Med., 11 (5):551-5,2005).

The cited experimental works have demonstrated that the intracerebral administration of NGF is effective in preventing, or at least delaying, neuronal death caused by the aforesaid pathologies. Moreover, none of these studies showed any side effects in animals. However, it must be noted that in all the aforesaid publications, the NGF was administered to the cerebral tissues via intracerebral injection.

European patent EP 0721343 (Syntex), for example, concerns NGF-based pharmaceutical formulations proposed for the treatment of the aforesaid pathologies and recalls that NGF-based preparations have the drawback of being poorly absorbed by the body if administered orally, and that the only solution possible was by parenteral administration, injection or infusion. Since the proposed NGF-based preparation is unable to pass through the blood-brain barrier, the document proposed - for a product administration that must reach the brain tissues - intraventricular injections or infusions through an intracerebroventricularly implanted cannula or through intracerebral implants of delayed release devices or pumps.

The PCT international patent applications published respectively with No. WO 98/48002 and WO 00/44396, of which one of the present inventors is the author, so far turn out to be the only patent documentation containing a description of NGF use for external ophthalmic application, such as in the form of eye-drops or as an ointment. The experimental work reported demonstrates how the topical administration of NGF can successfully solve ocular surface pathologies (cornea and conjunctiva) and, unexpectedly, also pathologies concerning internal ocular tissues. Therefore, NGF turned out to be suitable for successfully treating ophthalmic pathologies for which no prior effective therapies existed.

Although the therapeutic activity of NGF for central nervous system pathologies has already been reported in the literature, and specifically for AD and PD, no solution has so far been proposed for the problem of an easy administration of the active ingredient to the tissues concerned. In effect, the techniques employing intracerebral injections or the implanting of devices inside brain tissues such that the active ingredient is continually released *in situ -* as in the case of the devices described in the aforesaid European patent EP 0721343 - present the risk of various complications, reported in the literature, such as infections, haemorrhages and injuries of the anatomical structures during injection. These complications can occur even more frequently in the treatment of chronic pathologies, and can lead to the inapplicability of the therapy causing an inversion of the risk-benefit balance.

According to the present invention, it was surprisingly found that, by administering NGF in the form of eye-drops on the ocular surface, it is possible to obtain an increase in the levels of this neurotrophin in all the brain tissues, including cerebrospinal fluid. It was confirmed that NGF, when administered in the form of eye-drops, is able to pass through at the central nervous system level, and can exert *in situ* its own therapeutic capacities on brain diseases (neurodegenerative and ischemic ones), including, specifically, Alzheimer's Disease and Parkinson's Disease.

As will be illustrated in detail in the experimental report below, the passage of the NGF molecule from the eye surface on which it is administered, towards the brain tissues, was demonstrated by using both a autoradiographic method (Levi-Montalcini, R. & Aloe L., Proc. Natl. Sci. USA 82: 7111-7115,1985), and an immunoenzymatic dosage (Bracci-Laudiero, L. et al., Neurosci. Lett., 147: 9-12,1992). In particular, by applying the latter method on rats treated with a conjunctival instillation of NGF in a saline solution, it was found that, two hours after administration, there was an increase in NGF concentration in all the brain tissues examined. This increase in concentration gradually decreased to base levels after about 24 hours.

This effect allows NGF to perform a therapeutic action not only in internal ocular tissues not directly concerned by its surface administration, as had already been hypothesised previously, but also - and unexpectedly - in tissues of the brain region outside the eye, and thus, specifically, in the central nervous system. This aspect is innovative for brain pathologies, for which the therapeutic effectiveness of NGF had already been demonstrated, but there being at the time no pharmacological administration method available enabling an easy administration of the active ingredient with no danger or side effects for the patient.

It is obvious that the possibility of having an external topical administration of a therapeutic agent of an ophthalmic type, i.e. in the form of eye-drops or ointment, when the site of action desired for the active ingredient is the central nervous system, represents a considerable benefit compared to administration via the already known parenteral route and by intracerebral injection.

Therefore, the present invention specifically provides the use of nerve growth factor (NGF) for the production of an ophthalmic preparation for administration on the ocular surface, for the therapy and/or prophylaxis of pathologies of the central nervous system, wherein the said ophthalmic preparation is in the form of eye-drops and contains from 10 to 500 µg/ml of NGF.

The present invention also provides nerve growth factor (NGF) for use in the therapy and/or prophylaxis of pathologies affecting the central nervous system, wherein the NGF is in an ophthalmic preparation for administration on the ocular surface and wherein said ophthalmic preparation is in the form of eye-drops and contains from 10 to 500 µg/ml of NGF.

Specifically, the preparation according to the present invention can be indicated for the therapy and/or prophylaxis of pathologies affecting the encephalic structures, or for the therapy and/or prophylaxis of pathologies affecting the spinal chord. In both cases, the NGF-based ophthalmic preparation may be indicated for the therapy of pathologies of a post-traumatic nature, or of an infective, post-surgical, autoimmune, dystrophic, degenerative, ischemic or post-inflammatory nature. Among these, besides the pathologies that have been the main object of the research leading to the present invention, one can also include various forms of dementia of a non-degenerative type, such as those following repeated mechanical traumas and that are sometimes known as "boxer's dementia", or those following cerebrovascular disorders.

More specifically, the NGF-based ophthalmic preparation according to the present invention is indicated for the therapy and/or prophylaxis of neurodegenerative brain pathologies, among which, according to some forms of preferred realisation of the present invention, Parkinson's Disease and Alzheimer's Disease.

According to other embodiments of the present invention, the ophthalmic preparation may also be applied to the therapy and/or prophylaxis of neuronal degenerations of the corticospinal tract, of which amyotrophic lateral sclerosis constitutes another example on which NGF may have positive effects.

In the present description it must be noted that by "nerve growth factor" or "NGF" is meant any biologically active form of NGF, preferably but not exclusively subunit β, either natural or recombinant, but even hybrid or modified forms of NGF that bind to their corresponding receptor and preserve the bioavailability of NGF, or even NGF fragments or hybrids in which some amino acids have been eliminated or substituted, obviously on condition that the resulting product maintains a sufficient capacity to bind to the specific receptor. In this context, the present invention also includes, for example, the use of NGF-based proteins in conjunction with carrier proteins, such as transferrin, and compounds exerting an NGF-like action through NGF receptor bonds.

More specifically, the said NGF-based ophthalmic preparation is in the form of a solution or suspension (collyrium or eye-drops), ointment, gel or cream with a pharmaceutically acceptable ophthalmic carrier tolerated by the eye and compatible with the active ingredient. It is also possible to envisage particular forms of ophthalmic administration based on delayed release, as erodible ocular inserts or polymer membrane "reservoir" systems placed within the conjunctival sac. Alternatively, NGF, or a preparation containing it, may be added to a local bandage with a therapeutic contact lens.

As already noted and also confirmed by the experimental data reported below, external topical administration of NGF also turned out to be able to inhibit neuronal degeneration in animal models of Alzheimer's Disease and Parkinson's Disease. NGF administrations through applications on the ocular surface induces neuron survival and a behavioural improvement of the animal in animal models of Alzheimer's Disease and Parkinson's Disease, when there is a loss of brain neurons.

More specifically, the effects of the NGF administered as eye-drops in Parkinson's Disease are the following: 1) a decrease in the number of apoptotic neurons in the substantia nigra compacta (SNc); 2) an increase in the number of dopaminergic (DAergic) neurons in the SNc; 3) a decrease in the number of Lewy bodies (including the cytoplasmic types typical of Parkinson's Disease and which are considered a marker); 4) an increase in the number of neurons in other brain regions including the locus coeruleus, hypothalamus, the cerebral cortex and basal nucleus; 5) a decrease in the circular movement behaviour of rats (spatial disorientation with circular movement, another typical symptom of the disease).

The effects of NGF administered in the form of eye-drops in Alzheimer's Disease are the following: 1) a decrease in the number of apoptotic neurons in the hippocampus and cortical region; 2) an increase in the number of cholinergic neurons in the basal forebrain, hippocampus and cortical region; 3) a decrease in the number of amyloid plaques; 4) a decrease in Aβ42 peptide levels (a marker of Alzheimer's Disease, where Aβ represents amyloid protein); 5) a decrease in inflammation; 6) an increase in acetylcholine levels.

The possibility that NGF could exert a biological action on brain tissues following topical ophthalmic administration was hardly foreseeable, above all, in view of the fact that - as highlighted above - NGF is a molecule of considerable size (26,800 dalton) with a complex structure. In order for a molecule to have any action on brain tissues, then - once instilled on the eye surface - it must be able to pass through the eye tissues and reach the optic nerve and cerebrospinal fluid in order to exert its own biological activity on the brain region. In current practice, ocular administration is not used for treating brain pathologies. The foregoing is due to the fact that all the known studies concerning NGF use in brain pathologies have only employed intracerebral administration.

In actual fact, although having a complex structure and a high molecular weight, NGF has both hydrophilic and hydrophobic structural groups which enable it to pass through the homologous (lipid and hydrophilic) anatomical barriers. Moreover, a fundamental feature of NGF is that once it reaches the target organs even at very low, but biologically active, concentrations, it can stimulate the endogenous production of NGF by the tissue itself. The presence of an endogenous fraction of NGF is clearly borne out by the results of experiments (which will be illustrated below) on the passage of NGF through tissues. These results also show that a concentration gradient is not maintained from the external surface of the eye towards the brain, as had been hypothesised for a simple diffusion mechanism through tissues.

In order to produce the preparation according to the present invention, suitable procedures for NGF extraction and purification are reported in the aforesaid literature. The technique of Bocchini and Angeletti, briefly reported here, was used for experiments concerning the present invention. Submandibular glands of adult male mice are extracted in sterile conditions and the tissues thereof are homogenised, centrifuged and dialysed. The suspension is then made to pass through successive cellulose columns, whereon the NGF is adsorbed. The NGF is then eluted from the column by means of a 0.4 M sodium chloride buffer. The samples thus obtained are analysed spectrophotometrically at a wavelength of 280 nm to identify the NGF containing fractions. These fractions are dialysed and the NGF is lyophilised in sterile conditions and refrigerated at -20°C.

A medicinal product according to the present invention and suitable for ocular surface administration preferably contains, either alone or in association with one or more other active ingredients, between 10 and 500 µg/ml, and still more preferably in the range 200-250 µg/ml. A specific formulation for eye-drops may contain, for example, 200 µg/ml of NGF in a 0.9% sodium chloride physiological solution, or in a balanced saline solution (BSS^{®}): in both cases, the solution is isotonic with tear fluid and thus well tolerated by the eye. However, it is also possible to use hypotonic solutions.

The NGF contained in the saline solutions can be present either alone or in association with other biologically active molecules and/or conjugated with other carrier molecules (such as transferrin). In order to further enhance its passage through the ocular surface, other excipients selected from among those currently used in pharmaceutical techniques, such as in order to buffer solutions or suspensions to stabilise the active ingredient and make the preparation well tolerated, can be added. More specifically, the buffers should maintain the pH within the range 4-8, and preferably within the range pH=6.5 and pH=7.5. For example, the aforesaid sodium chloride solution may be buffered with any of the buffers well known in the pharmaceutical field as suitable for ophthalmic use, among which phosphate or trizma (tri-hydroxymethylaminomethane), in order to have a physiological pH of 7.0-7.4, at the same time maintaining physiological osmolarity (295-305 mOsm/l).

Tolerability may be further enhanced by using excipients such as polysorbate 80 (or Tween 80), dextran, polyethyleneglycol (e.g., PEG 400) and the like. The formulation can also contain viscosity-enhancing agents such as hyaluronic acid, methylcellulose, polyvinylalcohol, polyvinylpyrrolidon and the like, in order to enhance the bioavailability, stability and tolerability of the active ingredient. The bioavailability of NGF can be further enhanced by using substances that increase the corneal permeation of the drug, such as dimethylsulfoxide, taurocholates, membrane phospholipids and various surfactant agents suitable for ophthalmic use. Moreover, a preservative agent having antimicrobial activity can be added to the formulation in order to prevent contamination.

Agents like carboxymethylcellulose or the like can be added to products to be administered in the form of suspension. If it is desired to use the formulation as an ointment, gel or ophthalmic cream, the NGF carrier could be polyethylene glycol, polyacrylates, polyethylene oxides, fatty acids and alcohols or lanolin, paraffin and the like.

Some experimental results obtained within the frame of the present invention are reported below merely for exemplificative purposes.

### Studies on the passage of NGF from the eye surface to the brain

In a first set of tests to evaluate the passage of NGF intraocularly from the external surface, on which it is administered, to the brain tissues, the aforesaid autoradiographic method was used on a group of six rabbits. Each of the rabbits received one collyrium drop (50 µl) containing 10 µg of I¹²⁵ labelled NGF (concentration: 200 µg/ml) by instillation in the conjunctival fornix.

Murine NGF was used, purified according to the previously described procedure and subsequently conjugated to Na-I¹²⁵ (Amersham Italia, IMS30, 1mCi) according to the chloramine T method (Lapack P.A., Exp. Neurol., 124:1620, 1993). The quantity of labelled NGF was determined by chromatography by using a Sephadex G-25 column. The quantity of I¹²⁵ labelled product collectible by precipitation was between 90 and 95%, demonstrating that most of the radioactive product was bonded to NGF. The specific activity of NGF-I¹²⁵ was between 1 and 1.5 Ci/µmol.

Two hours following the administration of the labelled NGF, the animals were sacrificed and the brains dissected and fixed in 4% paraformaldehyde for 48 hours. Then, after incubation in 30% sucrose for 24 hours, the samples were cut with a cryostat to 15 µm thick sections, which were mounted on histology gelatinous slides, immersed in photographic emulsion (Ilford K2) and incubated for 4 weeks at 4°C. The sections were then dehydrated with ethanol, mounted with DPX after treatment with xylene and examined with a Zeiss optical microscope.

This experiment demonstrated that, once administered on the eye surface, the labelled NGF was able to penetrate into the eye and to bond with cells of various brain tissues (including the cortex, hippocampus, basal forebrain, substantia nigra, locus coeruleus, hypothalamus and basal nucleus) expressing the specific receptor.

In a second set of tests, using the aforesaid immunoenzymatic method, the quantitative levels of NGF in various brain tissues were determined following the administration of one drop of murine NGF in the conjunctival fornix. A total of 24 rats were used, six of which were sacrificed immediately in order to determine the base values of NGF concentration in the various brain tissues. The remaining animals were sacrificed 2 hours (6 rats), 6 hours (6 rats) and 24 hours (6 rats) after eye-drop administration.

In all the cases, the cerebrospinal fluid and brain were collected, and the different brain regions (including the cortex, hippocampus, basal forebrain, substantia nigra, locus coeruleus, hypothalamus and basal nucleus) were dissected. After weighing, the tissues were sonicated (using a Braun B Sonicator) in a buffered protein matrix containing protease inhibitors (extraction buffer). The homogenate thus obtained was centrifuged (10000 g for 20 minutes) and the supernatant was used in order to determine the NGF levels by the immunoenzymatic method (ELISA). The technique used is extremely sensitive and NGF-specific, and is able to detect concentrations up to 5 pg/ml. Goat anti-NGF polyclonal was used as a first antibody, diluted in a 0.05 M carbonate buffer at a pH of 9.6. As a control, to determine the unspecific signal, purified goat immunoglobulins were used.

The solutions with the primary antibody and with the control immunoglobulins were distributed in parallel on 96-well polystyrene plates. The plates were incubated for 12 hours at room temperature and then the unspecific sites were blocked with a carbonate buffer solution plus 1% BSA. After washing the plates with a solution of Tris-HCl 50 mM at pH 7.4, 200 mM NaCl, 0.5% gelatine and 0.1 % Triton X-100, the NGF samples and standard solutions were suitably diluted with a solution of Tris-HCl 100 mM at pH 7.2, 400 mM NaCl, 4 mM EDTA, 0.2 mM PMSE, 0.2 mM benzethonium chloride, 2 mM benzimidine, 40 U/ml aprotinin, 0.05% sodium azide, 2% BSA and 0.5% gelatine. After triplicate distribution of NGF samples and standard solutions in an amount of 50 µl/well, the plates were incubated with the secondary antibody: 4 mU/well of anti-β-NGF-galactoxidase (Boehringer, Mannheim, Germany) for 2 hours at 37°C. Then, after washing, 100 µl/well of a solution containing 4 mg of β-galactosil-chlorophenol red (Boehringer, Mannheim, Germany) per ml of a solution containing 100 mM HEPES, 150 mM NaCl, mM MgCl₂, 0.1% sodium azide and 1% BSA was distributed.

After incubation of the chromogen for two hours at 37°C, the optical density was determined at a wavelength of 575 nm by means of an ELISA reader (Dynatech). The concentration values of the NGF standards and samples were calculated after subtracting the background values due to unspecific bonds. The data expressed as pg/ml or as pg/gr refer to fresh weighed tissue. The results, summarised in Table 1 below, show how there is an increase in NGF levels in all the brain tissues 2 hours after eye-drop administration; these values remain high, albeit reduced, and then return to base levels after 24 hours.

**TABLE 1**

| NGF levels in various brain tissues after NGF administration in the form of eye-drops (NGF pg/g of tissue) | | | | | | | | |
|---|---|---|---|---|---|---|---|---|
| HOURS | cerebro-spinal fluid | cortex | hippocampus | basal forebrain | substantia nigra | locus coeru-leus | hypothalamus | basal nucleus |
| 0 | 20±7 | 70±12 | 135±32 | 102±23 | 98±34 | 75±36 | 170±45 | 112±24 |
| 2 | 90±15 | 120±17 | 176±14 | 153±34 | 178±25 | 154±71 | 286±71 | 165±31 |
| 6 | 530±59 | 312±52 | 212±23 | 264±68 | 329±60 | 312±79 | 396±54 | 254±42 |
| 24 | 30±12 | 81±20 | 127±21 | 120±31 | 135±59 | 91±84 | 198±38 | 131±39 |

### Studies on the effect of NGF administration in the form of eye-drops, in Parkinson's Disease

To assess the effectiveness of NGF administration on the ocular surface for the treatment of Parkinson's Disease, NGF in the form of eye-drops was administered in an animal model 4 times a day, at a concentration of 200 µg/ml diluted in a balanced saline solution.

The characteristics of Parkinson's Disease (PD) include bradykinesis, tremor at rest and motor disorders. The pathological features include mainly dopaminergic (DAergic) neuron degeneration in the substantia nigra compacta (SNc) and the presence of Lewy bodies. The degeneration of other systems has also been reported, including the locus coeruleus, hypothalamus, cerebral cortex, basal nucleus, the central and peripheral components of the autonomic nervous system. Parkinson Disease symptoms are seen when there is about an 80% depletion of DAergic neurons in the SNc. In the absence of biological markers, the predictability of an early dysfunction of the DAergic system is problematic. Although there are no spontaneous manifestations of Parkinson's Disease (PD) in animals, the many animal experimental models of PD have been used in order to obtain the same clinical features in animals. The techniques employing neurotoxins to produce injuries in the nigrostriatal dopaminergic system have a considerable selectivity and reproducibility. In this experiment, the neurotoxin used was 6-hydroxydopamine (6-OHDA).

The rats were anaesthetised by i.p. injection of hydrate chloride. Domaminergic lesions by stereotactical injection of 6-OHDA (Sigma), 8µg/3ml of saline solution in the body of the right medial forebrain (coordinates from Paxinos & Watson's atlas: AP 4.4 mm, L +1.2 mm and V 7.8 mm from the duramadre, incision 2.4 mm below the interaural line).

One month after the lesion, the circular movement behaviour was found in all the rats with D-amphetamine (with 5 mg/kg in saline solution) i.p. and apomorphine hydrochlorate (50µg/kg) subcutaneously. The circular movement behaviour was assessed by using an automatic rotormeter (Rotorcid, Cuba) and the number of complete circles of 360°/min was automatically recorded. Only those rats showing circular movement behaviour for at least 7. complete ipsilateral turns per minute with a D-amphetamine injection in 90 minutes, and 3.3 complete controlateral turns per minute in a period of 45 minutes with apomorphine were included in the experimental study.

NGF in the form of eye-drops was administered for 2 months, 4 times a day, at a concentration of 200 µg/ml in a balanced saline solution, in the conjunctival fornix of 10 rats, while only a balanced saline solution was administered to another group of 10 rats as controls.

At the end of the treatment period (two months), the behavioural test was carried out again, after which the animals were sacrificed, their brains removed and assessed histologically as well as immunohistochemically. The data obtained are summarised in Table 2 below.

**TABLE 2**

| Effect of NGF treatment in the form of eye-drops in an animal model of Parkinson's Disease | | | | | |
|---|---|---|---|---|---|
| | Number of apoptotic neurons in the SNc* | Number of dopaminergic neurons in the SNc** | Number of Lewy bodies*** | Number of neurons in other brain regions *** | Circular movement behaviour |
| 2 months after treatment with NGF | 60% decrease | 220% increase | 130% decrease | 95% increase | 70% decrease |

| | | | | | |
|---|---|---|---|---|---|
| * determined using the TUNEL method and anti-caspase 3 antibody; ** determined immunohistochemically; *** determined histologically | | | | | |

The experiment above shows that NGF in the form of eye-drops is able to: 1) decrease the number of apoptotic neurons in the substantia nigra compacta (SNc); 2) increase the number of dopaminergic (DAergic) neurons in the SNc; 3) decrease the number of Lewy bodies; 4) increase the number of neurons in other brain regions including the locus coeruleus, hypothalamus, cerebral cortex and basal nucleus; 5) reduce the circular movement behaviour of rats.

### Studies on the effect of NGF in the form of eye-drops for Alzheimer's Disease

To assess the effectiveness of NGF administration on the ocular surface for the treatment of Alzheimer's Disease, NGF in the form of eye-drops was administered in an animal model 4 times a day, at a concentration of 200 µg/ml diluted in a balanced saline solution.

In the experiment described, doubly transgenic mice expressing both human mutations APPswe and PS1 A246E (APP PS1 mice) were used. These mice develop high levels of highly fibrillogenic Aβ42 peptide which develops amyloid plaques starting from around 9 months of age. The first amyloid plaques are present in the subiculum (and in the caudal cortex), and these deposits later extend to the hippocampus and to all the cortical regions. This feature is in some way similar to the early stages of Alzheimer's Disease, where the amyloid plaques are also widely restricted to the medial temporal cortical structures.

NGF in the form of eye-drops was administered for 2 months, 4 times a day, at a concentration of 200 µg/ml in a balanced saline solution, in the conjunctival fornix of 10 male mice (9 months old), while only a balanced saline solutions was administered to another group of 10 mice (same age and gender) to be used as controls.

At the end of the treatment period (two months) the animals were sacrificed, their brains removed and evaluated histologically and immunohistochemically. The data obtained are summarised in Table 3 below.

**TABLE 3**

| Effect of NGF treatment in the form of eye-drops in an animal model of Alzheimer's Disease | | | | | | |
|---|---|---|---|---|---|---|
| | No. of apoptotic neurons in the hippocampus and cortex* | No. of cholinergic neurons ** | No. of amyloid plaques *** | Aβ42 peptide levels** | Microglia activated (GFAP+ and CD11b+) ** | Acetylcholin levels **** |
| 2 months after NGF treatment | 65% decrease | 190% increase | 60% decrease | 76% decrease | 50% decrease | 120% increase |

| | | | | | | |
|---|---|---|---|---|---|---|
| * determined with the TUNEL method and anti-caspase 3 antibody; ** determined immunohistochemically; *** determined by Congo red colouring; **** determined with specific ELISA. | | | | | | |

The above experiment shows that NGF in the form of eye-drops is able to: 1) decrease the number of apoptotic neurons in the hippocampus and cortical region; 2) increase the number of cholinergic neurons in the basal forebrain, hippocampus and cortical region; 3) reduce the number of amyloid plaques; 4) decrease the Aβ42 peptide levels; 5) reduce inflammation; 6) increase the acetylcholine levels.

The present invention has been disclosed with reference to some specific embodiments thereof, but it is to be understood that variations or modifications can be brought by persons skilled in the art without departing from the scope of the appended claims.

## Claims

1. Nerve growth factor (NGF) for use in the therapy and/or prophylaxis of pathologies affecting the central nervous system, wherein the NGF is in an ophthalmic preparation for administration on the ocular surface and wherein said ophthalmic preparation is in the form of eye-drops and contains from 10 to 500 µg/ml of NGF.

2. Nerve growth factor for use according to claim 1, wherein the said ophthalmic preparation is indicated for the therapy and/or prophylaxis of pathologies affecting the encephalic structures.

3. Nerve growth factor for use according to claims 1 or 2, wherein the said ophthalmic preparation is indicated for the therapy and/or prophylaxis of pathologies affecting the spinal cord.

4. Nerve growth factor for use according to any one of claims 1 -3, wherein the said pathologies are pathologies of post-traumatic, infective, post-surgical, autoimmune, dystrophic, degenerative, ischemic and post-inflammatory origin.

5. Nerve growth factor for use according to claims 1 or 2, wherein the said ophthalmic preparation is indicated for the therapy and/or prophylaxis of neurodegenerative cerebral pathologies.

6. Nerve growth factor for use according to any one of claims 1-3, wherein the said ophthalmic preparation is indicated for the therapy and/or prophylaxis of neuronal degenerations of the corticospinal tract.

7. Nerve growth factor for use according to claim 5, wherein the said ophthalmic preparation is indicated for the therapy and/or prophylaxis of Alzheimer's Disease or Parkinson's Disease.

8. Nerve growth factor for use according to any one of claims 1-7, wherein the said ophthalmic preparation is in the form of a solution or suspension (eye-drops), ointment, gel or cream with a pharmaceutically acceptable ophthalmic carrier, or in the form of an erodible ocular insert or polymeric membrane "reservoir" system to be placed in the conjunctival sac, or is added to a local bandage with a therapeutic contact lens.

9. Nerve growth factor for use according to any one of claims 1-8, wherein the said eye-drops contain 200-250 µg/ml of NGF.

10. Nerve growth factor for use according to any one of claims 1-9, wherein the NGF in the said ophthalmic preparation is in combination with one or more active ingredients and/or conjugated with a carrier molecule.

11. Nerve growth factor for use according to any one of claims 1-10, wherein the said NGF is of murine or human origin, or it is recombinant human NGF or a compound exerting an NGF-like action through a bond with NGF receptors.

12. Use of nerve growth factor (NGF) for the production of an ophthalmic preparation for administration on the ocular surface, for the therapy and/or prophylaxis of pathologies affecting the central nervous system, wherein the said ophthalmic preparation is in the form of eye-drops and contains from 10 to 500 µg/ml of NGF.

13. Use according to claim 12, wherein the said ophthalmic preparation is indicated for the therapy and/or prophylaxis of Alzheimer's Disease or Parkinson's Disease.

## Patentansprüche

1. Nervenwachstumsfaktor (NGF) zur Verwerdung in der Therapie und/oder Prophylaxe von Pathologien, die das zentrale Nervensystem betreffen, wobei der NGF in einem ophthalmischen Präparat zur Verabreichung an die Augenoberfläche vorliegt und wobei das ophthalmische Präparat in der Form von Augentropfen vorliegt und 10 bis 500 µg/ml NGF enthält.

2. Nervenwachstumsfaktor zur Verwendung gemäß Anspruch 1, wobei das ophthalmische Präparat für die Therapie und/oder Prophylaxe von Pathologien, die enzephalische Strukturen betreffen, indiziert ist.

3. Nervenwachstumsfaktor zur Verwendung gemäß Ansprüchen 1 oder 2, wobei das ophthalmische Präparat für die Therapie und/oder Prophylaxe von Pathologien, die das Rückenmark betreffen, indiziert ist.

4. Nervenwachstumsfaktor zur Verwendung gemäß einem der Ansprüche 1-3, wobei die Pathologien Pathologien mit posttraumatischem, infektiösem, postchirurgischem, autoimmunem, dystrophischem, degenerativem, ischämischem oder postentzündlichem Ursprung sind.

5. Nervenwachstumsfaktor zur Verwendung gemäß Ansprüchen 1 oder 2, wobei das ophthalmische Präparat für die Therapie und/oder Prophylaxe von neurodegenerativen Zerebralpathologien indiziert ist.

6. Nervenwachstumsfaktor zur Verwendung gemäß einem der Ansprüche 1-3, wobei das ophthalmische Präparat für die Therapie und/oder Prophylaxe von neuronalen Degenerationen des Kortikospinaltrakts indiziert ist.

7. Nervenwachstumsfaktor zur Verwendung gemäß Anspruch 5, wobei das ophthalmische Präparat für die Therapie und/oder Prophylaxe der Alzheimer-Erkrankung oder der Parkinson-Erkrankung indiziert ist.

8. Nervenwachstumsfaktor zur Verwendung gemäß einem der Ansprüche 1-7, wobei das ophthalmische Präparat in der Form einer Lösung oder Suspension (Augentropfen), Salbe, eines Gels oder einer Creme mit einem pharmazeutisch verträglichen ophthalmischen Träger oder in der Form eines abbaubaren Augeneinsatzes oder eines in der Bindehauttasche zu platzierenden Polymermembran-"Reservoir" systems vorliegt, oder einem lokalen Verband mit einer therapeutischen Kontaktlinse zugegeben ist.

9. Nervenwachstumsfaktor zur Verwendung gemäß einem der Ansprüche 1-8, wobei die Augentropfen 200-250 µg/ml NGF enthalten.

10. Nervenwachstumsfaktor zur Verwendung gemäß einem der Ansprüche 1-9, wobei der NGF in dem ophthalmischen Präparat in Kombination mit einem oder mehreren Wirkstoffen und/oder mit einem Trägermolekül konjugiert vorliegt.

11. Nervenwachstumsfaktor zur Verwendung gemäß einem der Ansprüche 1-10, wobei der NGF von Mäuseursprung oder menschlichem Ursprung ist oder rekombinanter menschlicher NGF oder eine Verbindung ist, die durch eine Bindung mit NGF-Rezeptoren eine NGF-artige Wirkung ausübt.

12. Verwendung von Nervenwachstumsfaktor (NGF) bei der Herstellung eines ophthalmischen Präparats zur Verabreichung an die Augenoberfläche für die Therapie und/oder Prophylaxe von Pathologien, die das zentrale Nervensystem betreffen, wobei das ophthalmische Präparat in der Form von Augentropfen vorliegt und 10 bis 500 µg/ml NGF enthält.

13. Verwendung gemäß Anspruch 12, wobei das ophthalmische Präparat für die Therapie und/oder Prophylaxe der Alzheimer-Erkrankung oder der Parkinson-Erkrankung indiziert ist.

## Revendications

1. Facteur de croissance du tissu nerveux (NGF) pour utilisation dans la thérapie et/ou la prophylaxie de pathologies affectant le système nerveux central, où le NGF est une préparation ophtalmique pour administration sur la surface oculaire et où ladite préparation ophtalmique est sous la forme de gouttes ophtalmiques et contient de 10 à 500 µg/ml de NGF.

2. Facteur de croissance du tissu nerveux pour utilisation selon la revendication 1, où ladite préparation ophtalmique est indiquée pour la thérapie et/ou la prophylaxie de pathologies affectant les structures encéphaliques.

3. Facteur de croissance du tissu nerveux pour utilisation selon les revendications 1 ou 2, où ladite préparation ophtalmique est indiquée pour la thérapie et/ou la prophylaxie de pathologies affectant la moelle épinière.

4. Facteur de croissance du tissu nerveux pour utilisation selon l'une quelconque des revendications 1 à 3, où lesdites pathologies sont des pathologies d'origine post-traumatique, infectieuse, postchirurgicale, auto-immune, dystrophique, dégénérative, ischémique et post-inflammatoire.

5. Facteur de croissance du tissu nerveux pour utilisation selon les revendications 1 ou 2, où ladite préparation ophtalmique est indiquée pour la thérapie et/ou la prophylaxie de pathologies cérébrales neurodégénératives.

6. Facteur de croissance du tissu nerveux pour utilisation selon l'une quelconque des revendications 1 à 3, où ladite préparation ophtalmique est indiquée pour la thérapie et/ou la prophylaxie de dégénérescences neuronales du tractus corticorachidien.

7. Facteur de croissance du tissu nerveux pour utilisation selon la revendication 5, où ladite préparation ophtalmique est indiquée pour la thérapie et/ou la prophylaxie de la maladie d'Alzheimer ou de la maladie de Parkinson.

8. Facteur de croissance du tissu nerveux pour utilisation selon l'une quelconque des revendications 1 à 7, où ladite préparation ophtalmique est sous la forme d'une solution ou suspension (gouttes ophtalmiques), d'une pommade, d'un gel ou d'une crème avec un véhicule ophtalmique pharmaceutiquement acceptable, ou sous la forme d'un système « réservoir » à membrane polymère ou d'insert oculaire érodable destiné à être placé dans le sac conjonctival, ou est ajoutée à un pansement local avec une lentille de contact thérapeutique.

9. Facteur de croissance du tissu nerveux pour utilisation selon l'une quelconque des revendications 1 à 8, où lesdites gouttes ophtalmiques contiennent 200 à 250 µg/ml de NGF.

10. Facteur de croissance du tissu nerveux pour utilisation selon l'une quelconque des revendications 1 à 9, où le NGF dans ladite préparation ophtalmique est en combinaison avec une ou plusieurs substances actives et/ou conjugué avec une molécule de véhicule.

11. Facteur de croissance du tissu nerveux pour utilisation selon l'une quelconque des revendications 1 à 10, où ledit NGF est d'origine murine ou humaine, ou est un NGF humain recombinant ou un composé exerçant une action de type NGF par l'intermédiaire d'une liaison avec des récepteurs NGF.

12. Utilisation de facteur de croissance du tissu nerveux (NGF) pour la production d'une préparation ophtalmique pour administration sur la surface oculaire, pour la thérapie et/ou la prophylaxie de pathologies affectant le système nerveux central, où ladite préparation ophtalmique est sous la forme de gouttes ophtalmiques et contient de 10 à 500 µg/ml de NGF.

13. Utilisation selon la revendication 12, où ladite préparation ophtalmique est indiquée pour la thérapie et/ou la prophylaxie de la maladie d'Alzheimer ou de la maladie de Parkinson.
